# EUROPÄISCHE PATENTSCHRIFT

(11) **EP 2 869 756 B1**
(45) Veröffentlichungstag und Bekanntmachung des Hinweises auf die Patenterteilung: **27.09.2017**
(21) Anmeldenummer: 13732359.8
(22) Anmeldetag: 21.06.2013
(51) Int. Cl.: A61B 5/02, G01M 3/16

(54) **FEUCHTIGKEITSSENSOR ZUR ÜBERWACHUNG EINES ZUGANGS ZU EINEM PATIENTEN UND VERFAHREN ZUR HERSTELLUNG DES FEUCHTIGKEITSSENSORS**
HUMIDITY SENSOR FOR MONITORING AN ACCESS TO A PATIENT, AND METHOD FOR PRODUCING THE HUMIDITY SENSOR
CAPTEUR D'HUMIDITÉ POUR SURVEILLER UN ACCÈS À UN PATIENT ET PROCÉDÉ POUR LA FABRICATION DU CAPTEUR D'HUMIDITÉ

(30) Priorität: 09.07.2012 DE 102012013471; 09.07.2012 US 201261669162 P
(43) Veröffentlichungstag der Anmeldung: 13.05.2015
(73) Patentinhaber: Fresenius Medical Care Deutschland GmbH, 61352 Bad Homburg (DE)
(72) Erfinder: WÜPPER, Andreas, 64572 Büttelborn (DE)
(74) Vertreter: Oppermann, Frank
(86) Internationale Anmeldenummer: PCT/EP2013/001843
(87) Internationale Veröffentlichungsnummer: WO 2014/008981

(56) Entgegenhaltungen:
- WO-A1-2006/008866
- WO-A1-2010/091852
- WO-A1-2011/116943
- DE-A1-102010 024 654
- US-A- 4 298 855
- US-A1- 2002 198 483

## Beschreibung

Die Erfindung betrifft einen Feuchtigkeitssensor zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung. Darüber hinaus betrifft die Erfindung ein Verfahren zur Herstellung eines Feuchtigkeitssensors zur Überwachung eines Patientenzugangs.

Auf dem Gebiet der Medizintechnik sind verschiedene Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicher zu stellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verzügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird. Bei der akuten Dialyse auf Intensivstationen wird hingegen ein zentralvenöser Katheter am Hals des Patienten eingesetzt.

Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Es sind Vorrichtungen zur Überwachung eines Gefäßzugangs bekannt, die über eine Vorrichtung zum Detektieren von Feuchtigkeit verfügen, um an der Punktionsstelle das Austreten von Blut erkennen zu können. Die bekannten Feuchtigkeitssensoren sind als ein auf die Punktionsstelle aufzulegendes Pad ausgebildet, das aus einem saugfähigen Material besteht, auf das eine elektrisch leitende Struktur aufgebracht ist oder in das eine elektrisch leitende Struktur eingebettet ist. Die elektrisch leitende Struktur besteht aus im Abstand zueinander angeordneten Leiterbahnen, die in dem oder auf dem Trägermaterial angeordnet sind. Eine Benetzung des Trägermaterials mit Flüssigkeit führt zu einer Veränderung des elektrischen Widerstands zwischen den Leiterbahnen.

Die bekannten Feuchtigkeitssensoren werden über eine Verbindungsleitung mit einer Einrichtung verbunden, die eine Veränderung des elektrischen Widerstandes zwischen den Leiterbahnen erkennt.

Eine elektrisch leitende Struktur, die zwei an den Enden offene Leiterbahnen umfasst, die im Abstand zueinander angeordnet sind, ist insofern nachteilig, als die Auswerteinrichtung weder eine Beschädigung der Leiterbahnen, noch einen fehlerhaften Anschluss des Feuchtigkeitssensors erkennen kann, da eine Unterbrechung der Leiterbahn oder Verbindungsleitung und eine fehlerhafte Anschlusskontaktierung nicht zu einer Veränderung des elektrischen Widerstands führt. Daher ist es bekannt, die Enden der beiden Leiterbahnen über einen Abschlusswiderstand miteinander zu verbinden. Wenn der mit der Auswerteinrichtung gemessene elektrische Widerstand des Feuchtigkeitssensors dem elektrischen Widerstand des Abschlusswiderstands der Leiterbahnen entspricht, kann davon ausgegangen werden, dass der Feuchtigkeitssensor intakt und korrekt an die Auswerteinrichtung angeschlossen ist.

Es sind Feuchtigkeitssensoren bekannt, die aus unterschiedlichen Trägermaterialien bestehen. Als Trägermaterial finden beispielsweise textile Materialien Verwendung. Das Aufbringen des Abschlusswiderstands auf das Trägermaterial erweist sich fertigungstechnisch als problematisch. Es sind daher Feuchtigkeitssensoren bekannt, die nicht über einen Abschlusswiderstand verfügen, sondern an einen externen Abschlusswiderstand angeschlossen werden. Das Anschließen externer Abschlusswiderstände ist jedoch aufwendig.

Die WO 2011/116943 beschreibt einen Feuchtigkeitssensor, der als ein Gewebe aus nicht-leitfähigen Kettfäden und nicht-leitfähigen Schussfäden sowie leitfähigen Kettfäden und leitfähigen Schussfäden ausgebildet ist, die derart angeordnet sind, dass sie eine elektrisch leitende Struktur bilden, die eine erste und eine zweite Leiterbahn umfasst. Die Enden der beiden Leiterbahnen sind als Anschlusskontakte ausgebildet, die an einer Lasche seitlich versetzt zueinander angeordnet sind. An die Lasche des Feuchtigkeitssensors wird das Anschlussstück eines Verbindungskabels angeschlossen, mit dem der Feuchtigkeitssensor mit der Auswerteinrichtung elektrisch verbunden wird. Die Auswerteinrichtung misst den elektrischen Widerstand zwischen dem einen Paar von Anschlusskontakten des Feuchtigkeitssensors, während das andere Paar von Anschlusskontakten über einen Abschlusswiderstand miteinander verbunden ist, der in das Anschlussstück integriert ist.

Ein Feuchtigkeitssensor, an den ein externer Abschlusswiderstand angeschlossen wird, ist auch aus der DE 10 2010 024 654 A1 bekannt. Der Feuchtigkeitssensor weist ein flexibles Trägermaterial auf, auf das eine elektrisch leitende Struktur aus Leiterbahnen aufgebracht ist.

Ein Feuchtigkeitssensor, der eine auf einer Trägerschicht angeordnete Leiterbahnanordnung umfasst, ist aus der DE 10 2010 023 132 A1 bekannt. Die beiden Leiterbahnen des Feuchtigkeitssensors sind durch eine Kontaktschicht miteinander verbunden, die ihren elektrischen Widerstand im befeuchteten Zustand ändert.

Die US 4 298 855 A beschreibt einen Feuchtigkeitssensor mit vier feuchtigkeitsempfindlichen Widerständen, die eine Messbrücke bilden, um eine von der Temperatur unabhängige Messung der Feuchtigkeit vornehmen zu können. Die feuchtigkeitsempfindlichen Widerstände bestehen aus *polymer films,* die auf eine Platine aufgebracht sind. Die Platine weist auch Leiterbahnen auf, die eine elektrisch leitende Struktur bilden.

Aus der WO 2010/091852 ist ein Feuchtigkeitssensor bekannt, bei dem die Leiterbahnstruktur und der Abschlusswiderstand mittels Siebdrucktechnik auf ein Trägermaterial aufgebracht sind. Für die Leiterbahnstruktur wird eine silberhaltige Druckpaste und für die Widerstandsstruktur eine kohlehaltige Druckpaste verwendet. Das Aufbringen des Abschlusswiderstands mittels Drucktechnik ist zwar auch bei einem Feuchtigkeitssensor aus einem textilen Material grundsätzlich möglich, erweist sich jedoch wegen der zusätzlichen Verfahrensschritte, die der Druckprozess erfordert, als nachteilig.

Der Erfindung liegt die Aufgabe zugrunde, einen in großen Stückzahlen kostengünstig herzustellenden Feuchtigkeitssensor zur Überwachung eines Patientenzugangs zu schaffen. Eine Aufgabe der Erfindung ist auch ein Verfahren zur kostengünstigen Herstellung des Feuchtigkeitssensors in großen Stückzahlen anzugeben.

Die Lösung dieser Aufgaben erfolgt erfindungsgemäß mit den Merkmalen der unabhängigen Patentansprüche. Die Gegenstände der Unteransprüche betreffen vorteilhafte Ausführungsformen der Erfindung.

Der erfindungsgemäße Feuchtigkeitssensor weist ein auf die Haut des Patienten aufzulegendes Trägermaterial mit einer elektrisch leitenden Struktur auf, die im Abstand zueinander angeordnete Leiterbahnen umfasst, die über einen elektrischen Abschlusswiderstand miteinander verbunden sind. Das Trägermaterial des erfindungsgemäßen Feuchtigkeitssensors kann unterschiedlich beschaffen sein. Es kann sich um ein textiles oder nicht textiles Trägermaterial handeln. Vorzugsweise sollte das Trägermaterial aber saugfähig sein.

Der erfindungsgemäße Feuchtigkeitssensor zeichnet sich dadurch aus, dass der elektrische Abschlusswiderstand eine elektrisch leitfähige Folie ist, die in einem Abschnitt des Trägermaterials aufgebracht ist, an dem elektrische Kontaktbereiche für den Anschluss des elektrischen Abschlusswiderstands an die Leiterbahnen ausgebildet sind. Die elektrischen Kontaktbereiche für den Anschluss des Abschlusswiderstands können auf der dem Patienten zugewandten Unterseite oder der dem Patienten abgewandten Oberseite des Feuchtigkeitssensors vorgesehen sein. Die Kontaktbereiche müssen aber auf der gleichen Seite vorgesehen sein, um die elektrisch leitende Folie aufbringen zu können.

Der erfindungsgemäße Feuchtigkeitssensor hat den Vorteil, dass ein externer Abschlusswiderstand nicht erforderlich ist. Dadurch wird der Anschluss des Feuchtigkeitssensors vereinfacht. Der erfindungsgemäße Feuchtigkeitssensor lässt sich in großen Stückzahlen zusammen mit dem Abschlusswiderstand einfach herstellen, indem die leitfähige Folie als Folienbahn während des Produktionsprozesses aufgebracht wird.

Nach dem Aufbringen der Folienbahn können die Feuchtigkeitssensoren zusammen mit dem Abschlusswiderstand vereinzelt werden, indem die Feuchtigkeitssensoren zusammen mit dem Abschlusswiderstand aus der gemeinsamen Materialbahn ausgeschnitten oder ausgestanzt werden.

Die Vorteile des erfindungsgemäßen Feuchtigkeitssensors kommen insbesondere dadurch zum Tragen, dass das Trägermaterial ein textiles Material, insbesondere ein Gewebe ist, auf das sich pastöse Massen als Abschlusswiderstand nur schwer aufbringen lassen.

Bei einer bevorzugten Ausführungsform, mit der sich das Herstellungsverfahren besonders einfach durchführen lässt, sind die elektrisch leitfähige Folie und das Trägermaterial mit einem elektrisch leitfähigen Kleber verklebt. Elektrisch leitfähige Folien und elektrisch leitfähige Kleber gehören zum Stand der Technik.

Eine alternative Ausführungsform sieht anstelle einer Verklebung mit einem leitfähigen Kleber die Verbindung von elektrisch leitfähiger Folie und Trägermaterial mit einer sich über die elektrisch leitfähige Folie erstreckenden Deckfolie vor, die auf das Trägermaterial aufgebracht ist. Die Verbindung von Deckfolie und Trägermaterial erfordert bei der alternativen Ausführungsform nicht die Verwendung eines elektrisch leitfähigen Klebers.

Die elektrisch leitende Struktur kann unterschiedlich ausgebildet sein. Bei einer bevorzugten Ausführungsform weist die elektrisch leitende Struktur eine erste Leiterbahn und eine zweite Leiterbahn auf, wobei die elektrischen Kontaktabschnitte für den Anschluss des elektrischen Abschlusswiderstands an dem einen Ende der ersten Leiterbahn und an dem einen Ende der zweiten Leiterbahn ausgebildet sind. Das andere Ende der ersten Leiterbahn und das andere Ende der zweiten Leiterbahn sind vorzugsweise als elektrische Anschlusskontakte für ein elektrisches Anschlussstück zum Anschluss des Feuchtigkeitssensors an die Auswerterichtung ausgebildet. Vorzugsweise sind die elektrischen Kontaktabschnitte für den Abschlusswiderstand an der einen Seite und die elektrischen Anschlusskontakte für das Anschlussstück an der anderen Seite des Trägermaterials angeordnet. Dadurch ist es möglich, den Abschlusswiderstand dadurch zu
schaffen, dass die elektrisch leitfähige Folie auf die Seite des Feuchtigkeitssensors mit den Kontaktabschnitten aufgebracht wird.

Das Trägermaterial des Feuchtigkeitssensors ist vorzugsweise als ein Flächengebilde ausgebildet, wobei die elektrischen Kontaktabschnitte und die elektrischen Anschlusskontakte an einer Lasche des Flächengebildes angeordnet sind. Die Lasche erlaubt den einfachen Anschluss des Anschlussstücks einerseits und das Aufbringen der elektrisch leitfähigen Folie andererseits, da die Lasche räumlich von der elektrisch leitfähigen Struktur getrennt ist.

Eine besonders bevorzugte Ausführungsform sieht ein Trägermaterial aus nicht-leitfähigen Kettfäden und nicht-leitfähigen Schussfäden sowie aus leitfähigen Kettfäden und leitfähigen Schussfäden vor, wobei die elektrisch leitende Struktur aus leitfähigen Kettfäden und/oder Schussfäden ausgebildet ist. Bei dieser Ausführungsform kommen die Vorteile des erfindungsgemäßen Feuchtigkeitssensors besonders zum Tragen, da sich die Folienbahn aus der leitfähigen Folie einfach mit der Gewebebahn verbinden lässt. Die elektrisch leitfähige Folie kann jede Folie sein, die einen passenden elektrischen Widerstand hat. Als elektrisch leitfähige Folien sind beispielsweise die bekannten Carbonfolien oder Polymerfolien geeignet.

Der Abschlusswiderstand sollte einen Wert haben, der größer ist als der Widerstand des befeuchteten Feuchtigkeitssensors und kleiner als der Wert des Innenwiderstands der Auswerteinrichtung einschließlich des Widerstands des Verbindungskabels. Wenn der Sensor unabhängig von der Luftfeuchtigkeit der Umgebung und auf einzelne Flüssigkeitstropfen ansprechen soll, hat sich in der Praxis ein Wert für den Abschlusswiderstands von 100 kOhm bis 1000 kOhm als vorteilhaft erwiesen.

Das Verfahren zur Herstellung des Feuchtigkeitssensors zeichnet sich dadurch aus, dass in einem ersten Verfahrensschritt eine Gewebebahn aus nicht-leitfähigen Kettfäden und nicht-leitfähigen Schussfäden sowie leitfähigen Kettfäden und leitfähigen Schussfäden gewebt wird, wobei die leifähigen und nicht-leitfähigen Kett- und Schussfäden derart angeordnet werden, dass die leitfähigen und nicht-leitfähigen Kett- und Schussfäden eine elektrisch leitende Struktur in dem Gewebe bilden. Ein derartiges Webverfahren gehört zum Stand der Technik. Dieses Webverfahren ist in der WO 2011/116943 beschrieben.

An den Webprozess schließt sich zur Herstellung des Abschlusswiderstands nur ein zusätzlicher Verfahrensschritt an, der sich leicht in den Herstellungsprozess integrieren lässt. In diesem Verfahrensschritt wird eine Folienbahn aus einer elektrisch leitfähigen Folie, die vorzugsweise eine selbstklebende Folie ist, auf die Gewebebahn aufgebracht. Dabei ist die Breite der Folienbahn kleiner als die Breite der Gewebebahn, wobei die Folienbahn auf den Abschnitt des Trägermaterials aufgebracht wird, an dem die elektrischen Kontaktabschnitte für den elektrischen Abschlusswiderstand ausgebildet sind. Anschließend werden die einzelnen Feuchtigkeitssensoren vereinzelt, in dem die Sensoren in der vorgesehenen Kontur aus der Materialbahn ausgeschnitten oder ausgestanzt werden. Das erfindungsgemäße Verfahren kann noch weitere Verfahrensschritte umfassen, zu denen beispielsweise das Waschen, das Imprägnieren, das Aufbringen einer Adhäsionsschicht und das Abdecken mit einer Abdeckschicht, beispielsweise ein Silikonpapier, oder dergleichen zählen.

Im Folgenden wird ein Ausführungsbeispiel der Erfindung unter Bezugnahme auf die Figuren im Einzelnen erläutert.

Es zeigen:
- Fig. 1: die wesentlichen Komponenten einer Blutbehandlungsvorrichtung, die über eine Vorrichtung zur Überwachung eines Gefäßzugangs verfügt,
- Fig. 2: den Feuchtigkeitssensor mit der elektrisch leitenden Struktur in schematischer Darstellung,
- Fig. 3A: ein erstes Ausführungsbeispiel der Anschlusslasche des Feuchtigkeitssensors in der Draufsicht,
- Fig. 3B: die Anschlusslasche von Fig. 3A in der Unteransicht,
- Fig. 4A: ein zweites Ausführungsbeispiel der Anschlusslasche in der Draufsicht,
- Fig. 4B: die Anschlusslasche von Fig. 4A in der Unteransicht,
- Fig. 5: ein drittes Ausführungsbeispiel der Anschlusslasche in der Draufsicht,
- Fig. 6: ein Ausführungsbeispiel der Anschlusslasche des Feuchtigkeitssensors zusammen mit einem ersten Ausführungsbeispiel des Abschlusswiderstands,
- Fig. 7: die Anschlusslasche von Fig. 6 mit einem zweiten Ausführungsbeispiel des Abschlusswiderstands,
- Fig. 8: eine Darstellung der wesentlichen Verfahrensschritte zur Herstellung des erfindungsgemäßen Feuchtigkeitssensors,
- Fig. 9: eine Darstellung des Verfahrensschritts des Aufbringens des Abschlusswiderstands auf den Feuchtigkeitssensor und
- Fig. 10: ein alternatives Ausführungsbeispiel der Anschlusslasche des Feuchtigkeitssensors

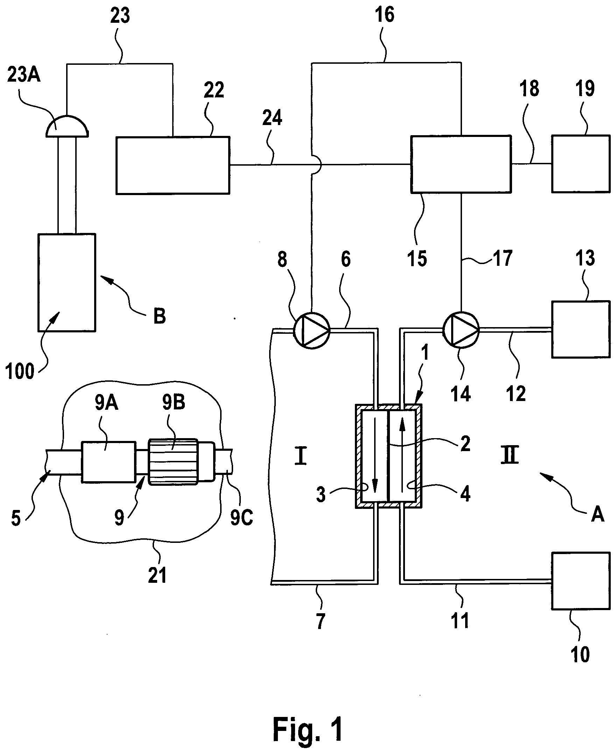

Fig. 1 zeigt die wesentlichen Komponenten einer Blutbehandlungsvorrichtung, insbesondere Hämodialysevorrichtung A für die Akutdialyse, die über eine Vorrichtung B zur Überwachung eines Gefäßzugangs, insbesondere eines Gefäßzugangs mit einem zentralvenösen Katheter verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. Der Gefäßzugang zum Patienten erfolgt mit einem zentralvenösen Katheter 5, der am Hals des Patienten angeschlossen wird. Der zentralvenöse Katheter 5 ist Teil des nur andeutungsweise dargestellten extrakorporalen Blutkreislaufs I, der die Blutkammer 3 des Dialysators 1 einschließt und Schlauchleitungen 6, 7 umfasst. Zum Fördern des Bluts im extrakorporalen Kreislauf ist ein Blutpumpe 8 vorgesehen.

Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 8, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Die nur schematisch dargestellte Überwachungsvorrichtung B dient bei dem vorliegenden Ausführungsbeispiel zur Überwachung einer Luer-Lock-Verbindung 9 mit den Verbindungsteilen 9A und 9B zum Anschluss des zentralvenösen Katheters 5 an eine Schlauchleitung 9C des extrakorporalen Blutkreislaufs I. Die Überwachungsvorrichtung B verfügt über eine Vorrichtung 100 zur Erkennung von Feuchtigkeit, die an der Schlauchverbindungsstelle 21 angeordnet wird. Dieser Feuchtigkeitssensor 100 ist in Fig. 1 nur schematisch dargestellt. Darüber hinaus verfügt die Überwachungsvorrichtung B über eine Auswerteinrichtung 22, die über eine Verbindungsleitung 23 mit dem Feuchtigkeitssensor 100 elektrisch verbunden ist. Die Verbindungsleitung 23 ist mit einem elektrischen Verbindungsstück 23A an den Feuchtigkeitssensor 100 angeschlossen.

Über eine Datenleitung 24 ist die Auswerteinrichtung 22 mit der zentralen Steuereinheit 15 der Dialysevorrichtung A verbunden. Für den Fall, dass Blut aus der Schlauchverbindungsstelle 21 austritt und den Feuchtigkeitssensor 100 befeuchtet, erzeugt die Auswerteinrichtung 22 der Überwachungsvorrichtung B ein Steuersignal, das die zentrale Steuereinheit 15 über die Datenleitung 24 empfängt, die einen Eingriff in die Blutbehandlung vornimmt. Die Steuereinheit 15 stoppt die Blutpumpe 8 und erzeugt ein Alarmsignal, sodass die Alarmeinheit 19 einen akustischen und/oder optischen und/oder taktilen Alarm gibt.

Nachfolgend wird der erfindungsgemäße Feuchtigkeitssensor 100 im Einzelnen beschrieben. Der Feuchtigkeitssensor weist als Trägermaterial ein Mehrlagengewebe auf, das aus elektrisch leitfähigen und elektrisch nicht-leitfähigen Kett- und Schussfäden (Monofilamente, Carbonfasern, versilbertes Polyamidgarn) besteht. Der Feuchtigkeitssensor 1 weist eine der Haut des Patienten zugewandte Unterseite und eine der Haut des Patienten abgewandte Oberseite auf. Er weist einen mittleren Bereich 100A auf, an den sich ein linker und ein rechter Schenkel 100B, 100C anschließen, die einen kreisförmigen Ausschnitt 100D umfassen. An der den Schenkeln gegenüberliegenden Seite schließt sich an den mittleren Bereich 100A eine Anschlusslasche 100E an.

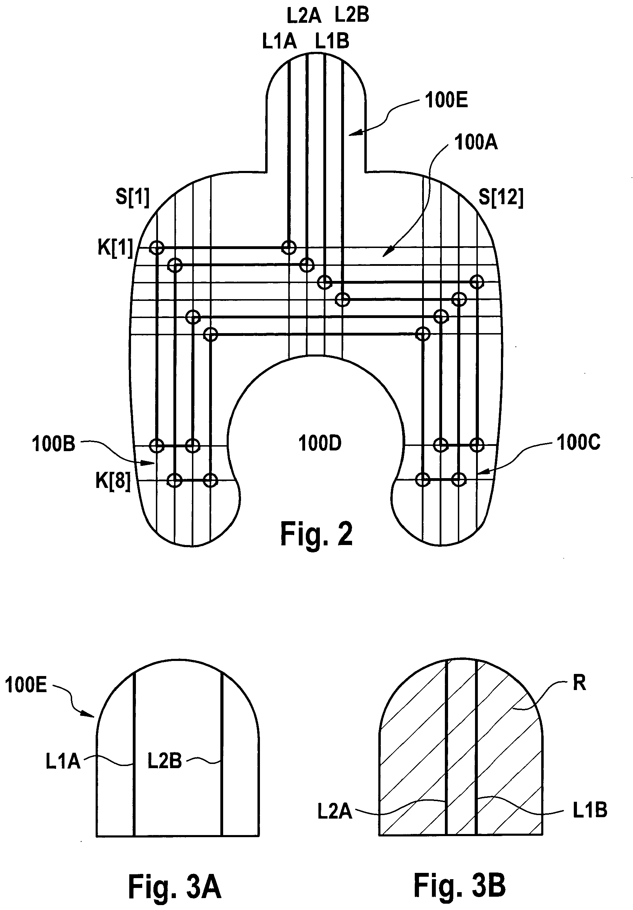

Die elektrisch leitfähigen und elektrisch nicht-leitfähigen Kett- und Schussfäden sind in dem Mehrlagengewebe derart angeordnet, dass eine elektrisch leitende Struktur ausgebildet wird. Die elektrisch leitende Struktur besteht aus orthogonal zueinander verlaufenden Kett- und Schussfäden, die elektrisch leitfähig sind. In Fig. 2 sind die elektrisch leitfähigen Kett- und Schussfäden mit K[1] bis K[8] bzw. S[1] bis S[12] bezeichnet. Die Kreuzungspunkte der Kett- und Schussfäden sind in Fig. 2 durch Kreise dargestellt. Der in Fig. 2 gezeigte Feuchtigkeitssensor ist eine bevorzugte Ausführungsform zur Überwachung der venösen und/oder arteriellen Nadel bei der chronischen Hämodialyse.

Die elektrisch leitende Struktur L1, L2 umfasst zwei Leiterbahnen L1 und L2. Der Anfang jeder Leiterbahn ist mit "A" und das Ende der Leiterbahnen mit "B" bezeichnet. Die erste Leiterbahn L1 verläuft von der Anschlusslasche 100E über den mittleren Bereich 100A zu dem linken Schenkel 100B und von dem linken Schenkel über den mittleren Bereich zu dem rechten Schenkel 100C und von dem rechten Schenkel über den mittleren Bereich wieder zu der Lasche 100E. Die zweite Leiterbahn L2 verläuft von der Lasche 100E über den mittleren Bereich 100A zu dem linken Schenkel 100B und von dem linken Schenkel über mittleren Bereich zu dem rechten Schenkel und von dem rechten Schenkel über den mittleren Bereich zu der Lasche. Beide Leiterbahnen L1 und L2 verlaufen parallel und im Abstand zueinander. Sie weisen jeweils mehrere orthogonal zueinander stehende Abschnitte auf.

Die einen Enden L1A und L2B der ersten und zweiten Leiterbahn L1, L2 bilden zwei äußere Anschlusskontakte an der Oberseite der Lasche 100E (Fig. 3A), während die anderen Enden L1B und L2A der ersten und zweiten Leiterbahn L1, L2 zwei innere Kontaktabschnitte an der Unterseite der Lasche 100E bilden (Fig. 3B). Diese Kontaktabschnitte sind in den Figuren 3A und 3B schematisch dargestellt

Die beiden inneren Kontaktabschnitte L1B und L2A werden mit einem Abschlusswiderstand R elektrisch verbunden, der in den Figuren nicht dargestellt ist. Bei dem Abschlusswiderstand R handelt es sich um eine leitfähige Folie, die auf die Ober- oder Unterseite der Lasche 100E aufgebracht wird.

Bei dem Ausführungsbeispiel ist die elektrisch leitfähige Folie R auf die Unterseite der Lasche 100E aufgebracht, mit der die Kontaktabschnitte L2A und L1B der Leiterbahnen L1, L2 miteinander verbunden sind. Diese Folie ist in Fig. 3B durch eine Schraffur gekennzeichnet.

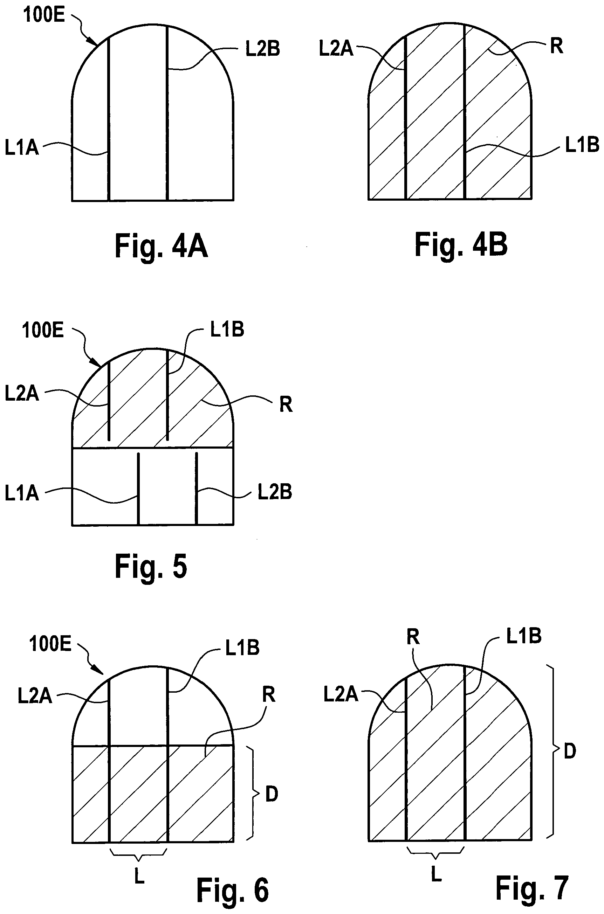

Die Figuren 4A und 4B zeigen die Ober- und Unterseite eines zweiten Ausführungsbeispiels der Lasche 100E, das sich von dem ersten Ausführungsbeispiel durch die Anordnung der Kontaktabschnitte und Anschlusskontakte an der Ober- oder Unterseite der Lasche unterscheidet, wobei sich an der Oberseite der Lasche 100E ein äußerer und ein innerer Anschlusskontakt L1A, L2B (Fig. 4A) und an der Unterseite der Lasche ein äußerer und ein innerer Kontaktabschnitt L2A, L1B befinden (Fig. 4B). Die leitfähige Folie R ist bei diesem Ausführungsbeispiel auf die Unterseite der Lasche 100E mit den Kontaktabschnitten L2A, L1B aufgebracht (Fig. 4B). Die leitfähige Folie R ist wieder durch schraffierte Linien dargestellt.

Fig. 5 zeigt ein weiteres Ausführungsbeispiel der Lasche 100E, bei dem sich die beiden Anschlusskontakte und die beiden Kontaktabschnitte nur an der Oberseite der Lasche 100E befinden. Beispielsweise befinden sich die Anschlusskontakte L1A, L2B an der dem mittleren Abschnitt 100A zugewandten Innenseite und die Kontaktabschnitte L2A, L1B an der Außenseite der Lasche, wobei die Anschlusskontakte und Kontaktabschnitte seitlich zueinander versetzt sind. Bei diesem Ausführungsbeispiel ist die leitfähige Folie R auf die Oberseite der Anschlusslasche 100E aufgebracht, wobei sich die Folie aber nur über die äußere Hälfte der Anschlusslasche erstreckt, in der sich die Kontaktabschnitte L2A, L1B befinden. Die leitfähige Folie R ist durch schraffierte Linien dargestellt.

Fig. 6 zeigt die Kontaktabschnitte L2A, L1B an der Oberseite einer Lasche 100E, die mit der elektrisch leitfähigen Folie R elektrisch miteinander verbunden sind. Die elektrisch leitende Folie R erstreckt sich nur über die untere Hälfte der Anschlusslasche. Der Abstand zwischen den parallelen streifenförmigen Kontaktabschnitten L2A, L1B ist mit L und die Breite der leitfähigen Folie R mit D bezeichnet. Über den Abstand L der Kontaktabschnitte und über die Breite D der Folie kann der elektrische Widerstand R₀ des Abschlusswiderstands R bei gegebenem Flächenwiderstand der Folie in gewissen Grenzen eingestellt werden. Der Widerstand R₀ ist bei gegebenem Flächenwiderstand der Folie proportional zum Abstand L und umgekehrt proportional zur Breite D. Die leitfähige Folie R ist wieder durch schraffierte Linien dargestellt.

Der elektrische Widerstand R₀ des Abschlusswiderstands sollte zwischen 100 kOhm und 1000 kOhm liegen. Daraus ergibt sich ein Flächenwiderstand für die elektrisch leitfähige Folie, die zwischen 2x10⁴ Ohm/sq. und 5x10⁷ Ohm/sq liegt. Dieser Wert entspricht dem Flächenwiderstand der bekannten elektrisch leitfähigen Polymerfolien (dissipative Polymere). Aber auch die bekannten elektrisch leitfähigen Carbonfolien, die als Schutz vor elektrostatischer Aufladung eingesetzt werden, können als Abschlusswiderstand verwendet werden.

Bei einer bevorzugten Ausführungsform werden die elektrisch leitfähigen Folien mit einem elektrisch leitfähigen Kleber auf die Anschlusslasche 100E des Feuchtigkeitssensors 1 aufgeklebt, wobei die Folie einen Teil oder die gesamte Fläche der Lasche bedecken kann. Fig. 7 zeigt ein Ausführungsbeispiel, bei dem die elektrisch leitfähige Folie R die gesamte Fläche der Lasche 100E des Feuchtigkeitssensors bedeckt.

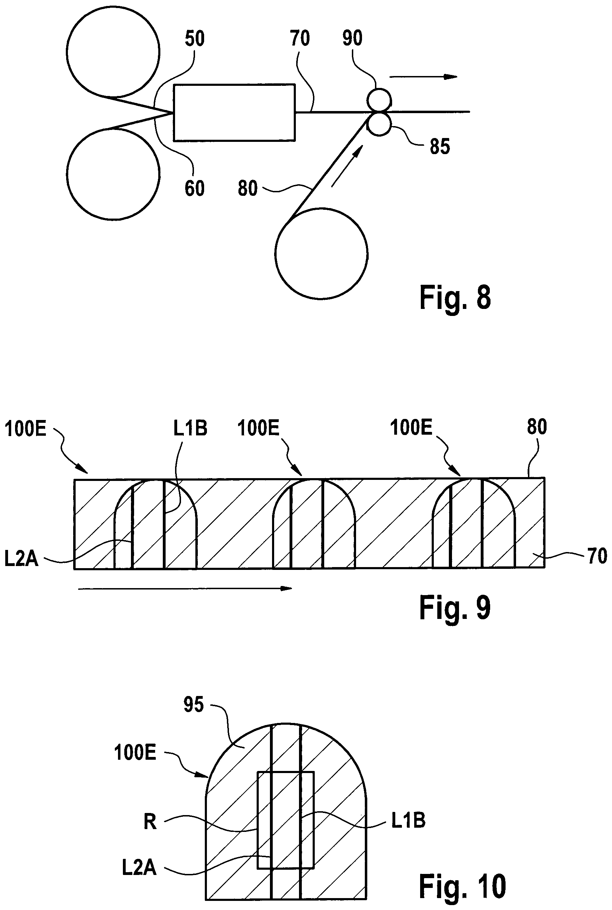

Fig. 8 zeigt in schematischer Darstellung den Verfahrensschritt zur Herstellung der Gewebebahn 70 aus Kett- und Schussfäden 50, 60 auf der sich eine Vielzahl von in Reihen und Spalten angeordneten Feuchtigkeitssensoren befinden, die jeweils eine elektrisch leitfähige Struktur aufweisen. In einem dem Webprozess nachfolgenden Verfahrensschritt wird auf die Ober- bzw. Unterseite der Laschen eine Folienbahn 80 aus einer elektrisch leitfähigen Folie aufgebracht, wobei Gewebe und Folie mit einem elektrisch leitfähigen Kleber verklebt werden. Die Gewebebahn wird auf den Bereich der Lasche an der Ober- bzw. Unterseite aufgebracht, an dem sich die Kontaktabschnitte zum Anschluss des Abschlusswiderstands befinden. Beim Aufbringen der Folienbahn 80 auf die Gewebebahn 70 durchlaufen Folie und Gewebebahn zwei Andruckrollen 85, 90, mit der die erforderliche Anpresskraft auf die Bahnen ausgeübt werden.

Fig. 9 zeigt in schematischer Darstellung den Teil der Gewebebahn 70, an der die Folienbahn 80 auf die Oberseite der Gewebebahn aufgebracht wird. Die schraffiert dargestellte Folienbahn 80 deckt die Oberseite der Laschen 100E mit den Kontaktabschnitten L2A, L1B vollflächig ab. Die Laufrichtung der nicht dargestellten Andruckrollen, zwischen denen die Bahnen laufen, ist mit einem Pfeil gekennzeichnet.

Nach dem Verkleben von Folien- und Gewebebahn 70, 80 werden die Feuchtigkeitssensoren ausgeschnitten oder ausgestanzt, um dann konfektioniert zu werden. Das Herstellungsverfahren umfasst noch weitere dem Fachmann bekannte Verfahrensschritte, die aber in Fig. 8 nicht dargestellt sind.

Fig. 10 zeigt eine alternative Ausführungsform der Anschlusslasche 100E des Feuchtigkeitssensors, bei dem Gewebe und Folie mit einem elektrisch leitfähigen Kleber verklebt sind. Bei diesem Ausführungsbeispiel liegt die elektrisch leitfähige Folie R nur auf den Kontaktabschnitten L2A, L1B auf. Die elektrisch leitfähige Folie R wird mit einer Deckfolie 95 fixiert, die sich über die Ränder der elektrisch leitfähigen Folie R hinaus erstreckt. Die Deckfolie 95 ist an der Unterseite mit einer Klebe- oder Adhäsionsschicht versehen, so dass die Deckfolie zur Fixierung der elektrisch leitfähigen Folie an der Lasche 100E haftet.

## Patentansprüche

1. Feuchtigkeitssensor zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, wobei der Feuchtigkeitssensor ein auf die Haut des Patienten aufzulegendes Trägermaterial mit einer elektrisch leitenden Struktur aufweist, die im Abstand zueinander angeordnete Leiterbahnen (L1, L2) aufweist, die über einen elektrischen Abschlusswiderstand (R) miteinander verbunden sind, das Trägermaterial ein textiles Flächengebilde (80) ist, in das die elektrische leitende Struktur eingebettet ist, und
**dadurch gekennzeichnet, dass** der elektrische Abschlusswiderstand eine elektrisch leitfähige Folie (R) ist, die in einem Abschnitt des Trägermaterials (80) aufgebracht ist, an dem elektrische Kontaktabschnitte (L2A, L1B) für den Anschluss des elektrischen Abschlusswiderstands an die Leiterbahnen (L1, L2) ausgebildet sind.

2. Feuchtigkeitssensor nach Anspruch 1, **dadurch gekennzeichnet, dass** auf die leitfähige Folie (R) eine sich über die leitfähige Folie erstreckende Deckfolie (95) aufgebracht ist, wobei die Deckfolie (95) und das Trägermaterial (80) miteinander verklebt sind.

3. Feuchtigkeitssensor nach Anspruch 2, **dadurch gekennzeichnet, dass** die leitfähige Folie (R) und das Trägermaterial (80) mit einem leitfähigen Kleber miteinander verklebt sind.

4. Feuchtigkeitssensor nach einem der Ansprüche 1 bis 3, **dadurch gekennzeichnet, dass** die elektrisch leitende Struktur eine erste Leiterbahn (L1) und eine zweite Leiterbahn (L2) aufweist, wobei die elektrischen Kontaktabschnitte (L2A, L1B) für den Anschluss des elektrischen Abschlusswiderstands an dem einen Ende der ersten Leiterbahn (L1) und dem einen Ende der zweiten Leiterbahn (L2) ausgebildet sind.

5. Feuchtigkeitssensor nach Anspruch 4, **dadurch gekennzeichnet, dass** das andere Ende der ersten Leiterbahn (L1) und das andere Ende der zweiten Leiterbahn (L2) als elektrische Anschlusskontakte (L1A, L2B) für ein elektrisches Anschlussstück zum Anschluss des Feuchtigkeitssensors ausgebildet sind.

6. Feuchtigkeitssensor nach Anspruch 5, **dadurch gekennzeichnet, dass** die elektrischen Kontaktabschnitte (L2A, L1B) an der einen Seite des Trägermaterials (80) und die elektrischen Anschlusskontakte (L1A, L2B) an der anderen Seite des Trägermaterials (80) angeordnet sind.

7. Feuchtigkeitssensor nach Anspruch 5 oder 6, **dadurch gekennzeichnet, dass** das Trägermaterial (80) als ein Flächengebilde ausgebildet ist, wobei die elektrischen Kontaktabschnitte (L2A, L1B) und die elektrischen Anschlusskontakte (L1A, L2B) an einer Anschlusslasche (100E) des Flächengebildes angeordnet sind.

8. Feuchtigkeitssensor nach einem der Ansprüche 1 bis 7, **dadurch gekennzeichnet, dass** das Trägermaterial ein textiles Gewebe aus nicht-leitfähigen Kettfäden (K) und nicht-leitfähigen Schussfäden (S) sowie aus leitfähigen Kettfäden und leitfähigen Schussfäden ist, wobei die elektrische leitende Struktur aus leitfähigen Kettfäden und/oder Schussfäden ausgebildet ist.

9. Feuchtigkeitssensor nach einem der Ansprüche 1 bis 8, **dadurch gekennzeichnet, dass** die Leiterbahnen (L1, L2) in einer Mehrzahl von orthogonal zueinander verlaufenden Abschnitten nebeneinander liegenden angeordnet sind.

10. Feuchtigkeitssensor nach einem der Ansprüche 1 bis 9, **dadurch gekennzeichnet, dass** die elektrisch leitfähige Folie (R) eine Carbonfolie oder eine Polymerfolie ist.

11. Feuchtigkeitssensor nach einem der Ansprüche 1 bis 10, **dadurch gekennzeichnet, dass** der elektrische Abschlusswiderstand einen Widerstand hat, der zwischen 100 kOhm und 1000 kOhm liegt.

12. Verfahren zur Herstellung eines Feuchtigkeitssensors nach einem der Ansprüche 1 bis 11 mit folgenden Verfahrensschritten,
Weben einer Gewebebahn aus nicht-leitfähigen Kettfäden und nicht leitfähigen Schussfäden sowie leitfähigen Kettfäden und leitfähigen Schussfäden, wobei die leitfähigen und nicht-leitfähigen Kett- und Schussfäden derart angeordnet werden, dass die leitfähigen und nicht-leitfähigen Kett- und Schussfäden eine elektrisch leitende Struktur in dem Gewebe bilden,
Aufbringen einer Folienbahn aus einer elektrisch leitfähigen Folie auf den Abschnitt des Trägermaterials, an dem elektrische Kontaktabschnitte für den Anschluss des elektrischen Abschlusswiderstands ausgebildet sind, wobei die Breite der Folienbahn kleiner als die Breite der Gewebebahn ist und
Vereinzeln der einzelnen Feuchtigkeitssensoren.

13. Verfahren nach Anspruch 12, **dadurch gekennzeichnet, dass** die elektrisch leitfähige Folie eine selbstklebende Folie ist.

14. Verfahren nach Anspruch 13, **dadurch gekennzeichnet, dass** während des Aufbringens der Folienbahn auf die Gewebebahn die Folienbahn und die Gewebebahn zwischen zwei Andruckrollen verlaufen.

## Claims

1. Moisture sensor for monitoring an access to a patient for a system by which, via a flexible line, a liquid is fed to a patient and/or a liquid is fed out from the patient, the moisture sensor having a substrate material to be applied to the patient's skin, which has an electrically conductive structure which has conductor paths (L1, L2) arranged at a distance from one another which are connected together across a terminating resistor (R),
the substrate material being a textile planar material (80) in which the electrically conductive structure is embedded, and
**characterised in that** the terminating resistor is an electrically conductive film (R) which is applied in a section of the substrate material (80) on which are formed electrical contacting portions (L2A, L1 B) for connecting the terminating resistor to the conductor paths (L1, L2).

2. Moisture sensor according to claim 1, **characterised in that** a covering film (95) which extends over the conductive film is applied to the conductive film (R), the covering film (95) and the substrate material (80) being adhesive bonded together.

3. Moisture sensor according to claim 2, **characterised in that** the conductive foil (R) and the substrate material (80) are adhesive bonded together by a conductive adhesive.

4. Moisture sensor according to one of claims 1 to 3, **characterised in that** the electrically conductive structure has a first conductor path (L1) and a second conductor path (L2), with the electrical contacting portions (L2A, L1B) for the connection of the terminating resistor being formed at one end of the first conductor path (L1) and one end of the second conductor path (L2).

5. Moisture sensor according to claim 4, **characterised in that** the other end of the first conductor path (L1) and the other end of the second conductor path (L2) take the form of electrical connecting contacts (L1A, L2B) for an electrical connector for connecting up the moisture sensor.

6. Moisture sensor according to claim 5, **characterised in that** the electrical contacting portions (L2A, L1 B) are arranged on one side of the substrate material (80) and the electrical connecting contacts (L1A, L2B) are arranged on the other side of the substrate material (80).

7. Moisture sensor according to claim 5 or 6, **characterised in that** the substrate material (80) takes the form of a planar material, with the electrical contacting portions (L2A, L1B) and the electrical connecting contacts (L1A, L2B) being arranged on a connecting tongue (100E) belonging to the planar material.

8. Moisture sensor according to one of claims 1 to 7, **characterised in that** the substrate material is a woven textile material of non-conductive warp filaments (K) and non-conductive weft filaments (S) and conductive warp filaments and conductive weft filaments, the electrically conductive structure being formed by conductive warp filaments and/or weft filaments.

9. Moisture sensor according to one of claims 1 to 8, **characterised in that** the conductor paths (L1, L2) are laid out in a plurality of portions extending orthogonally to one another and situated next to one another.

10. Moisture sensor according to one of claims 1 to 9, **characterised in that** the electrically conductive film (R) is a carbon film or a polymer film.

11. Moisture sensor according to one of claims 1 to 10, **characterised in that** the resistance of the terminating resistor is between 100 kohms and 1000 kohms.

12. Method of producing a moisture sensor according to one of claims 1 to 11, having the following steps,
weaving of a web of woven material from non-conductive warp filaments and non-conductive weft filaments and conductive warp filaments and conductive weft filaments, the conductive and non-conductive warp and weft filaments being so arranged that the said conductive and non-conductive warp and weft filaments form an electrically conductive structure in the woven material,
application of a web of an electrically conductive film to that section of the substrate material on which electrical contacting portions for the connection of the terminating resistor are formed, the width of the web of film being smaller than the width of the web of woven material and
separation of the individual moisture sensors.

13. Method according to claim 12, **characterised in that** the electrically conductive film is a self-adhesive film.

14. Method according to claim 13, **characterised in that** the web of film and the web of woven material extend between two pressure-applying rollers during the application of the web of film to the web of woven material.

## Revendications

1. Capteur d'humidité pour surveiller un accès à un patient pour un dispositif avec lequel un fluide est acheminé à un patient et/ou un fluide est évacué du patient par une conduite souple, dans lequel
le capteur d'humidité présente un matériau de support à poser sur la peau du patient comportant une structure électriquement conductrice qui présente des pistes conductrices espacées les unes des autres (L1, L2) qui sont reliées les unes aux autres par une résistance de terminaison électrique (R),
le matériau de support est une structure textile plane (80) dans laquelle la structure électriquement conductrice est incorporée, et
**caractérisé en ce que**
la résistance de terminaison électrique est une feuille électriquement conductrice (R) qui est appliquée dans une section du matériau de support (80) sur lequel des sections de contact électrique (L2A, L1B) sont formées pour le raccordement de la résistance de terminaison électrique aux pistes conductrices (L1, L2).

2. Capteur d'humidité selon la revendication 1, **caractérisé en ce que** sur la feuille conductrice (R) est appliquée une feuille de recouvrement (95) s'étendant sur la feuille conductrice, dans lequel la feuille de recouvrement (95) et le matériau de support (80) sont collés l'un à l'autre.

3. Capteur d'humidité selon la revendication 2, **caractérisé en ce que** la feuille conductrice (R) et le matériau de support (80) sont collés l'un à l'autre avec un adhésif conducteur.

4. Capteur d'humidité selon l'une des revendications 1 à 3, **caractérisé en ce que** la structure électriquement conductrice présente une première piste conductrice (L1) et une deuxième piste conductrice (L2), dans lequel les sections de contact électrique (L2A, L1B) sont formées pour le raccordement de la résistance de terminaison électrique à une extrémité de la première piste conductrice (L1) et à une extrémité de la deuxième piste conductrice (L2).

5. Capteur d'humidité selon l'une la revendication 4, **caractérisé en ce que** l'autre extrémité de la première piste conductrice (L1) et l'autre extrémité de la deuxième piste conductrice (L2) sont formées comme des contacts de raccordement électrique (L1A, L2B) pour un connecteur pour le raccordement du capteur d'humidité.

6. Capteur d'humidité selon la revendication 5, **caractérisé en ce que** les sections de contact électrique (L2A, L1B) sont disposées sur un côté du matériau de support (80) et les contacts de raccordement électrique (L1A, L2B) sont disposés sur l'autre côté du matériau de support (80).

7. Capteur d'humidité selon la revendication 5 ou 6, **caractérisé en ce que** le matériau de support (80) est formé comme une structure plane, dans lequel les segments de contact électrique (L2A, L1B) et les contacts de raccordement électrique (L1A, L2B) sont disposés sur une patte de raccordement (100E) de la structure plane.

8. Capteur d'humidité selon l'une des revendications 1 à 7, **caractérisé en ce que** le matériau de support est un tissu textile en fils de chaîne non conducteurs (K) et en fils de trame non conducteurs (S) ainsi qu'en fils de chaînes conducteurs et en fils de trame conducteurs, dans lequel la structure électrique conductrice est formée de fils de chaîne et/ou de fils de trame conducteurs.

9. Capteur d'humidité selon l'une des revendications 1 à 8, **caractérisé en ce que** les pistes conductrices (L1, L2) sont disposées en une multiplicité des sections adjacentes évoluant sur le plan orthogonal les unes par rapport aux autres

10. Capteur d'humidité selon l'une des revendications 1 à 9, **caractérisé en ce que** la feuille électriquement conductrice (R) est une feuille de carbone ou une feuille de polymère.

11. Capteur d'humidité selon l'une des revendications 1 à 10, **caractérisé en ce que** la résistance de terminaison conductrice a une résistance qui se situe entre 100 kOhm et 1000 kOhm.

12. Procédé de fabrication d'un capteur d'humidité selon l'une des revendications 1 à 11, comportant les étapes de procédé suivantes :
tissage d'une bande de tissu en fils de chaîne non conducteurs et en fils de trame non conducteurs ainsi qu'en fils de chaîne conducteurs et en fils de trame conducteurs, dans lequel des fils de chaîne et des fils de trame conducteurs et non conducteurs sont disposés de telle sorte que les fils de chaîne et les fils de trame conducteurs et non conducteurs forment une structure électriquement conductrice dans le tissu,
application d'une bande de feuille constituée d'une feuille électriquement conductrice sur la section du matériau de support sur laquelle des sections de contact électrique sont formées pour le raccordement de la résistance de terminaison électrique, dans lequel la largeur de la bande de feuille est inférieure à la largeur de la bande de tissu et
séparation des capteurs d'humidité individuels.

13. Procédé selon la revendication 12, **caractérisé en ce que** la feuille électriquement conductrice est une feuille autocollante.

14. Procédé selon la revendication 13, **caractérisé en ce que**, pendant l'application de la bande de feuille sur la bande de tissu, la bande de feuille et la bande de tissu passent entre deux rouleaux de compression.
